# EUROPÄISCHE PATENTSCHRIFT

(11) **EP 0 742 206 B1**
(45) Veröffentlichungstag und Bekanntmachung des Hinweises auf die Patenterteilung: **29.09.1999**
(21) Anmeldenummer: 96105756.9
(22) Anmeldetag: 12.04.1996
(51) Int. Cl.: C07C 407/00, C07C 409/24

(54) **Percarbonsäurelösungen mit verbesserter Stabilität im Kontakt mit Edelstahl**
Percarboxylic acid solutions with improved stability in presence steel
Solutions d'acides percarboxyliques ayant une meilleure stabilité en présence d'acier

(30) Priorität: 12.05.1995 DE 19517465
(43) Veröffentlichungstag der Anmeldung: 13.11.1996
(73) Patentinhaber: Degussa-Hüls Aktiengesellschaft, 60287 Frankfurt am Main (DE)
(72) Erfinder: Thiele, Georg, Dr., 63452 Hanau (DE); Täubl, Peter, 9721 Weissenstein 186 (AT)

(56) Entgegenhaltungen:
- EP-A- 0 626 371
- US-A- 2 347 434
- US-A- 3 168 554
- US-A- 3 442 937
- PATENT ABSTRACTS OF JAPAN vol. 017, no. 017 (C-1016), 13.Januar 1993 & JP-A-04 243861 (NIPPON PEROXIDE CO LTD), 31.August 1992,

## Beschreibung

Die Erfindung betrifft ein Verfahren zur Herstellung von Percarbonsäurelösungen mit verbesserter Stabilität im Kontakt mit Edelstahl. Bei den Percarbonsäurelösungen handelt es sich um organische Lösungen, vorzugsweise aber um wäßrige Lösungen, insbesondere sogenannte Gleichgewichtspercarbonsäurelösungen, wobei die Percarbonsäure 1 bis 12 C-Atome und eine oder zwei Peroxycarboxylgruppen aufweist.

Percarbonsäurelösungen, insbesondere wäßrige Lösungen, welche eine wasserlösliche niedere Percarbonsäure enthalten, finden vielseitige Anwendung, etwa in Wasch-, Bleich- und Reinigungsmitteln sowie in mikrobiozid wirksamen Zusammensetzungen für Desinfektionszwecke im industriellen aber auch im häuslichen Bereich. Percarbonsäurelösungen in wasserarmen oder wasserfreien organischen Lösungsmitteln werden als Oxidationsmittel in der chemischen Synthese eingesetzt.

Zur Herstellung einer Percarbonsäurelösung wird die Carbonsäure in An- oder Abwesenheit eines organischen Lösungsmittels mit Wasserstoffperoxid, üblicherweise unter Verwendung einer wäßrigen Wasserstoffperoxidlösung, in Gegenwart eines stark sauren Katalysators umgesetzt. Sofern die Gleichgewichtseinstellung nicht durch externe Maßnahmen, etwa eine azeotrope Entwässerung, beeinflußt wird, verläuft die Umsetzung bis zur Gleichgewichtseinstellung, und das erhaltene Reaktionsgemisch wird als Gleichgewichtspercarbonsäurelösung bezeichnet. Eine derartige Gleichgewichtspercarbonsäurelösung enthält außer der Percarbonsäure somit ein Lösungsmittelsystem, worunter die nicht-umgesetzte Carbonsäure, Wasser - aus dem wäßrigen Wasserstoffperoxid, Reaktionswasser sowie gegebenenfalls zugesetztes Wasser - sowie nicht-umgesetztes Wasserstoffperoxid und, sofern anwesend, wasserlösliche organische Lösungsmittel verstanden werden. Die zum Erreichen der Gleichgewichtseinstellung erforderliche Zeit hängt von der Menge und der Säurestärke des zugesetzten Katalysators ab; in der Praxis werden daher üblicherweise Schwefelsäure oder Salpetersäure als Katalysator verwendet.

Aufgrund ihrer Brand- und Explosionsgefahr werden niedere Percarbonsäuren überwiegend in Form von Lösungen gehandhabt, jedoch kommt es auch in derartigen Lösungen bei erhöhter Temperatur, erhöhtem pH-Wert und insbesondere in Anwesenheit katalytisch wirksamer Schwermetallionen zu Zersetzungserscheinungen, wodurch der Percarbonsäuregehalt gemindert wird. Da stabilitätsbeeinträchtigende Faktoren bei der Herstellung, Lagerung und Handhabung von Percarbonsäurelösungen in der Praxis nie ausgeschlossen werden können, werden bei und/oder nach deren Herstellung ein oder mehrere Stabilisatoren, vorzugsweise synergistisch wirksame Kombinationen, in einer Menge von üblicherweise unter 0,1 Gew.-%, bezogen auf die Lösung, zugesetzt. Bekannte Stabilisatoren sind beispielsweise Dipicolinsäure (US-Patent 2,609,391), Pyrophosphat oder Pyrophosphorsäure (US-Patent 2,347,434) sowie eine Kombination der genannten beiden Stoffklassen (DE-Patent 43 17 420). Gemäß US-Patent 2,590,856 lassen sich verdünnte wäßrige Percarbonsäurelösungen durch Zugabe von 100 bis 1000 ppm eines polymeren Phosphats mit einem Molverhältnis von R₂O zu P₂O₅ von nicht größer als 1,7 zu 1, wobei R Alkalimetall, Ammonium oder Wasserstoff bedeutet, stabilisieren.

Percarbonsäurelösungen zeigen gegenüber zahlreichen metallischen Werkstoffen, vor allem Edelstahl, eine unerwünschte Korrosionswirkung, die neben der Schädigung des Werkstoffs zu einem Herauslösen von Metallionen aus dem Werkstoff und damit einer Kontamination der Percarbonsäurelösung führt. Die durch diese Metallionen katalysierte Zersetzung der Percarbonsäure kann durch die gebräuchlichen Stabilisatoren nicht verhindert werden und zudem kann es durch die freigesetzte Wärme zu einer gefährlichen, sich selbst beschleunigenden Zersetzung kommen. Die Lagerung und der Transport von Percarbonsäurelösungen, insbesondere wäßrigen Percarbonsäurelösungen, in Edelstahlbehältern führt demnach zu einem erhöhten Sicherheitsrisiko, weshalb bisher für den genannten Zweck praktisch ausschließlich Kunststoffbehälter zum Einsatz kamen.

Aus dem US-Patent 3,890,165 ist bekannt, daß sich Metalloberflächen, welche mit Persauerstoffverbindungen, wie Peressigsäurelösungen, in Kontakt kommen, durch Behandlung mit einer Polyphosphorsäurelösung passivieren lassen. Eine derartige Passivierung reicht aber für eine dauerhafte Lagerung, insbesondere bei größerem Materialdurchsatz, ohne Zunahme der Zersetzungsneigung nicht aus.

Nach E. Mücke, Pharmazie 34 (1979) 573 kann eine 0,2 gew.-%ige wäßrige Peressigsäurelösung im Kontakt mit Eisen durch die Zugabe von 1 Gew.-% Na₂H₂P₂O₇ oder von 1 Gew.-% einer Mischung aus gleichen Teilen Na₂H₂P₂O₇ und NaH₂PO₄ für die Dauer eines Tages stabilisiert werden. Nach V.B. Rudak et al., Zh. Prikl. Khim. (Leningrad) 55 (1983) 2128-2130 läßt sich die durch den Kontakt mit Edelstahl bedingte Zersetzung einer acetonischen Peressigsäurelösung durch den Zusatz von 1 Gew.-% Natriumpyrophosphat bei 20 °C für die Dauer von 7 Tagen verringern. In der JP-A 04 243 861 wird auf die verminderte Korrosionswirkung von Peressigsäurelösungen auf Eisen durch den Zusatz von Pyrophosphorsäure hingewiesen. Obgleich durch die zuvor genannten Stoffe die Zersetzung von Percarbonsäurelösungen in Gegenwart von Edelstahl zwar gemindert werden konnte, reicht diese Stabilisierung für die Praxis nicht aus. Demgemäß ist es Aufgabe der vorliegenden Erfindung, Percarbonsäurelösungen bereitzustellen, welche sich durch eine deutlich verminderte Korrosionswirkung und damit auch verminderte Zersetzung auszeichnen. Eine weitere Aufgabe richtet sich auf ein Verfahren zur Herstellung derartiger Percarbonsäurelösungen, dessen Wirtschaftlichkeit im wesentlichen jener bekannter Verfahren entspricht.

Gemäß US-Patent 3,168,554 lassen sich Percarbonsäuren durch ein Pyridin-2-methanol-Derivat stabilisieren. Eine Kombination aus einem Pyridin-2-methanol-Derivat und bestimmten Phosphorverbindungen, darunter Polyphosphorsäuren, führt zu einem synergistischen Stabilisierungseffekt.

Die JP-A 4243861 (Patent Abstracts of Japan Vol. 17, No. 17, C1016, 13.01.1993) lehrt, Pyrophosphorsäure zur Herstellung einer Peressigsäure mit verminderter Korrosivität und guter Stabilität als Katalysator zu verwenden. Andere Alternativen werden in diesem Dokument nicht aufgezeigt.

Gefunden wurde ein Verfahren zur Herstellung einer Percarbonsäurelösung mit verbesserter Stabilität im Kontakt mit Edelstahl, umfassend Umsetzung einer Carbonsäure mit 1 bis 12 C-Atomen und einer oder zwei Carboxylgruppen mit Wasserstoffperoxid in Gegenwart eines Lösungsmittelsystems und eines sauren Katalysators bei 0 bis 70 °C, das dadurch gekennzeichnet ist, daß man als Katalysator Polyphosphorsäure der Formel Hₙ₊₂PₙO₃ₙ₊₁, worin n für den mittleren Kondensationsgrad, der gleich oder größer 2,3 ist, steht, in einer Menge von 0,2 bis 10 Gew.-%, bezogen auf die Percarbonsäurelösung, verwendet.

Die Unteransprüche richten sich auf bevorzugte Ausführungsformen des erfindungsgemäßen Verfahrens.

Die erfindungsgemäße Menge an Polyphosphorsäure wird im Rahmen der Herstellung der Percarbonsäurelösungen zugesetzt. Wie nachfolgend noch aufgezeigt wird, erwies sich die Polyphosphorsäure als hochwirksamer Katalysator für die Bildung einer Percarbonsäure aus einer Carbonsäure und Wasserstoffperoxid. Zur erfindungsgemäßen Herstellung der Percarbonsäurelösungen können eine oder mehrere Polyphosphorsäuren verwendet werden. Da Polyphosphorsäure durch Eintragen von Phosphorpentoxid in Phosphorsäure gewonnen wird, liegen üblicherweise mehrere Polyphosphorsäuren mit unterschiedlichem Kondensationsgrad nebeneinander vor - siehe Gmelins Handbuch der anorganischen Chemie, 8. Auflage (1965), Band 16 P[C] 217-219 und 252-253. Bevorzugt zu verwendende Polyphosphorsäuren enthalten einen mittleren Kondensationsgrad von über 3, insbesondere über 4. Vorzugsweise enthalten die erfindungsgemäß hergestellten Percarbonsäurelösungen 0,5 bis 5 Gew.-%, insbesondere 1 bis 3 Gew.-% Polyphosphorsäure.

Erfindungsgemäße Percarbonsäurelösungen können eine oder mehrere aliphatische oder aromatische Percarbonsäuren enthalten, welche eine oder zwei Peroxycarboxylgruppen aufweisen; bevorzugte Lösungen enthalten eine aliphatische Monoperoxycarbonsäure mit 1 bis 6 C-Atomen, insbesondere Peressigsäure und Perpropionsäure, oder eine α,ω-Diperoxydicarbonsäure mit 4 bis 6 C-Atomen, also Diperoxybernsteinsäure, Diperoxyglutarsäure und

Diperoxyadipinsäure, wobei die jeweilige α,ω-Diperoxydicarbonsäure üblicherweise im Gemisch mit der Monoperoxy-α,ω-dicarbonsäure gleicher C-Atomzahl vorliegt. Bei den bevorzugten Percarbonsäurelösungen handelt es sich um wäßrige Systeme, insbesondere um sogenannte Gleichgewichtspercarbonsäurelösungen. Die Percarbonsäure und die ihr zugrundeliegende Carbonsäure derartiger wäßriger Lösungen müssen demgemäß eine ausreichende Wasserlöslichkeit aufweisen; dies trifft für die zuvor genannten Mono- und Diperoxycarbonsäuren mit bis zu 6 C-Atomen zu. Wäßrige Lösungen mit längerkettigen Percarbonsäuren enthalten als Lösungsvermittler zusätzlich ein ausreichend oxidationsstabiles wasserlösliches organisches Lösungsmittel. Erfindungsgemäße Percarbonsäurelösungen können auch wasserarm oder im wesentlichen wasserfrei sein, derartige Lösungen enthalten als Lösungsmittel ein oder mehrere organische Lösungsmittel, wie insbesondere aromatische Kohlenwasserstoffe und Carbonsäureester; derartige Lösungen können auch Mono- und Diperoxycarbonsäuren mit bis zu 12 C-Atomen enthalten. Das Lösungsmittelsystem wäßriger Percarbonsäurelösungen enthält demgemäß im wesentlichen nicht-umgesetzte wasserlösliche Carbonsäure, Wasser und nicht-umgesetztes Wasserstoffperoxid; das Lösungsmittelsystem von im wesentlichen rein organischen Percarbonsäurelösungen enthält ein oder mehrere organische Lösungsmittel und gegebenenfalls nicht-umgesetzte Carbonsäure.

Bevorzugt hergestellte wäßrige Gleichgewichtspercarbonsäurelösungen enthalten die Percarbonsäure, die ihr zugrundeliegende Carbonsäure, Wasserstoffperoxid und Wasser im Gleichgewichtszustand. Eine besonders bevorzugt hergestellte Percarbonsäurelösung besteht im wesentlichen aus 2,5 bis 43 Gew.-% Peressigsäure, 5 bis 74 Gew.-% Essigsäure, 1 bis 50 Gew.-% Wasserstoffperoxid, 10 bis 60 Gew.-% Wasser und 0,5 bis 10 Gew.-% Polyphosphorsäure sowie 0 bis 0,5 Gew.-% einer oder mehrerer üblicher Stabilisatoren.

Die Stabilität erfindungsgemäß hergestellter Percarbonsäurelösungen läßt sich durch die Anwesenheit eines oder mehrerer üblicher Stabilisatoren weiter steigern. Die Stabilisatorkonzentration liegt in der Regel unter 0,1 Gew.-%, bezogen auf die Lösung (unter 1000 ppm). Unter den als Stabilisatoren geeigneten Verbindungen kommen solche aus der Reihe der (a) Chelatkomplexbildner auf der Basis von Phosphonsäuren, wie Hydroxy-und Aminophosphonsäuren, Amino- und Hydroxycarbonsäuren, N-heterocyclischen Carbonsäuren sowie Salzen der genannten Säuren, (b) Pyrophosphorsäure und deren Salzen, (c) Radikalfänger auf der Basis alkylierter Hydroxyaromaten und (d) Zinnverbindungen infrage.

Unter den mit zweiwertigen Metallen Chelatkomplexe bildenden Phosphonsäuren sind beispielhaft zu nennen: Phosphonobernsteinsäure, 2-Phosphonobutan-1,2,4-tricarbonsäure; unter den Hydroxyphosphonsäuren 1-Hydroxyäthan-1,1-diphosphonsäure und Homologe mit 3 bis 6 C-Atomen; unter den Aminophosphonsäuren Aminotrimethylenphosphonsäure, Dimethylaminomethandiphosphonsäure, 1-Amino-1-phenylmethandiphosphonsäure, Aminoessigsäure-N,N-di(methylenphosphonsäure), Ethylendiamintetra(methylenphosphonsäure), Hexamethylendiamintetra(methylenphosphonsäure), Diethylentriaminpenta(methylenphosphonsäure), 3-Aminopropan-1-hydroxy-1,1-diphosphonsäure. Unter den Chelatkomplexbildnern auf der Basis von Aminocarbonsäuren sind Aminotriessigsäure und Ethylendiamintetraessigsäure, unter den Hydroxycarbonsäuren Zitronensäure und Poly-α-Hydroxyacrylsäure, unter den N-hetrocyclischen Carbonsäuren Picolinsäure, Dipicolinsäure, Chinolinsäure, 2,4-Lutidinsäure, Dinicotinsäure sowie die Alkalimetall- und Ammoniumsalze der unter die Gruppe (a) fallenden Verbindungen zu nennen. Bei den Zinn-Stabilisatoren handelt es sich vorzugsweise um Hydrate von Stannaten der Formel Me₂SnO₃, worin Me Alkalimetall oder Ammonium bedeutet; um Trübungserscheinungen zu vermeiden, werden Stannate nur in sehr geringer Menge, üblicherweise unter 200 ppm, berechnet als Sn eingesetzt. Erfindungsgemäß hergestellte wäßrige Gleichgewichtspercarbonsäurelösungen mit besonders guter Lagerstablilität und Korrosionsbeständigkeit gegenüber Edelstahl weisen Lösungen auf, welche als zusätzlichen Stabilisator Dipicolinsäure enthalten.

Die Herstellung einer erfindungsgemäßen Percarbonsäurelösung umfaßt die Umsetzung einer Carbonsäure mit 1 bis 12 C-Atomen und einer oder zwei Carboxylgruppen mit Wasserstoffperoxid in Gegenwart eines Lösungsmittelsystems und eines sauren Katalysators bei 0 bis 70 °C, die dadurch gekennzeichnet ist, daß man als Katalysator Polyphosphorsäure der Formel Hₙ₊₂PₙO₃ₙ₊₁, worin n für den mittleren Kondensationsgrad, der gleich oder größer 2,3 ist, steht, in einer Menge von 0,2 bis 10 Gew.-%, bezogen auf die Percarbonsäurelösung, verwendet.

Der erfindungsgemäße Katalysator Polyphosphorsäure weist gegenüber Phosphorsäure, Pyrophosphorsäure und Metaphosphorsäure (H₃P₃O₉) überraschenderweise eine wesentlich höhere katalytische Aktivität auf, so daß die Gleichgewichtseinstellung wesentlich rascher erfolgt als mit den zuvor genannten Phosphorsäuren. Im erfindungsgemäßen Verfahren wird eine solche Polyphosphorsäure eingesetzt, deren mittlerer (=durchschnittlicher) Kondensationsgrad n gleich oder größer 2,3, vorzugsweise größer 3 und insbesondere größer 4, ist. Eine Polyphosphorsäure mit n = 2,3 enthält beispielsweise 80,8 Gew.-% P₂O₅, eine solche mit n = 3 82,5 Gew.-% P₂O₃; handelsübliche Polyphosphorsäure mit einem P₂O₅-Gehalt um 85 Gew.-% ist als Katalysator für das erfindungsgemäße Verfahren sehr gut geeignet. Die Polyphosphorsäure kommt in jener Menge zum Einsatz, wie sie in der herzustellenden Percarbonsäurelösung enthalten sein soll. Zur Herstellung der bevorzugten wäßrigen Percarbonsäurelösungen werden eine ausreichend wasserlösliche Carbonsäure mit einer wäßrigen Wasserstoffperoxidlösung gemischt und in Gegenwart des erfindungsgemäßen Katalysators Polyphosphorsäure umgesetzt; zweckmäßigerweise erfolgt die Umsetzung bis zur Gleichgewichtseinstellung, und das hierbei erhaltene Reaktionsgemisch enthält außer der Percarbonsäure nicht-umgesetzte Carbonsäure, Wasser, nicht-umgesetztes Wasserstoffperoxid sowie Polyphosphorsäure. Die Umsetzung erfolgt zwischen 0 und 70 °C, bevorzugt wird jedoch 10 bis 30 °C.

Wasserstoffperoxid wird bei der Umsetzung üblicherweise in Form einer wäßrigen Wasserstoffperoxidlösung eingesetzt; der H₂O₂-Gehalt derartiger Lösungen liegt meistens zwischen 5 und 85 Gew.-%, vorzugsweise zwischen 30 und 70 Gew.-%. Soweit dies im Hinblick auf die gewünschte Percarbonsäurekonzentration erforderlich ist, kann dem Reaktionssystem als Komponente des Lösungsmittelsystems zusätzlich Wasser zugesetzt werden. Bei der Umsetzung mäßig wasserlöslicher Carbonsäuren und Percarbonsäuren können zusätzlich Alkohole als Lösungsvermittler mitverwendet werden, wobei allerdings zu berücksichtigen ist, daß in diesem Falle das Gleichgewichtssystem zusätzlich den entsprechenden Carbonsäureester sowie Percarbonsäureester enthält.

Zur Herstellung längerkettige oder aromatische Percarbonsäuren enthaltender wasserarmer oder wasserfreier Lösungen ist es zweckmäßig, die Carbonsäure in Gegenwart eines geeigneten oxidationsstabilen organischen Lösungsmittels oder Lösungsmittelsystems mit wäßrigem Wasserstoffperoxid in Gegenwart des Polyphosphorsäurekatalysators umzusetzen und das Gleichgewicht durch beispielsweise azeotropes Abdestillieren von Wasser auf die gewünschte Seite zu verschieben.

Der Katalysator Polyphosphorsäure kann entweder der umzusetzenden Carbonsäure oder dem wäßrigen Wasserstoffperoxid vor der Umsetzung zugesetzt werden; selbstverständlich ist es auch möglich, den Katalysator dem Gemisch aus Carbonsäure und wäßrigem Wasserstoffperoxid zuzusetzen. Sofern die Percarbonsäurelösung zusätzlich einen oder mehrere Stabilisatoren enthalten soll, werden diese entweder einem oder beiden der Reaktionspartner vor der Umsetzung oder dem Reaktionsgemisch während oder nach der Umsetzung zugesetzt.

Zur Herstellung wäßriger Gleichgewichtspercarbonsäurelösungen, etwa einer wäßrigen Peressigsäure oder Perpropionsäurelösung, werden eine Carbonsäure und eine wäßrige Wasserstoffperoxidlösung in einer solchen Menge umgesetzt, daß vorzugsweise eine Percarbonsäurelösung mit 5 bis 15 Gew.-% Percarbonsäure resultiert; je nach dem gewählten Molverhältnis Carbonsäure zu H₂O₂ zu Wasser ist es möglich, Lösungen herzustellen, welche bei gleicher Percarbonsäurekonzentration eine unterschiedliche H₂O₂-Konzentration aufweisen.

Es wurde gefunden, daß sich Percarbonsäurelösungen in üblichen Edelstahlbehältern mit wesentlich verminderter Zersetzungsgefahr lagern, transportieren und handhaben lassen, wenn die Percarbonsäure Polyphosphorsäure in anspruchsgemäßer Konzentration enthält. Derartige Lösungen führen auch nicht zur Korrosion des Behältermaterials.

Die erfindungsgemäßen Percarbonsäurelösungen zeichnen sich dadurch aus, daß der zu ihrer Herstellung eingesetzte Katalysator gleichzeitig Stabilisator und Korrosionsinhibitor ist. Die Herstellung der Percarbonsäurelösungen erfordert keine anderen Maßnahmen als jene, welche im Stand der Technik bekannt waren, ausgenommen die Verwendung der erfindungsgemäß erforderlichen Polyphosphorsäuren anstelle der bisher üblichen Mineralsäuren als Katalysator. Durch die Verwendung von Polyphosphorsäure als kombinierten Katalysator/Stabilisator, Korrosionsinhibitor und gegebenenfalls zusätzlich einen oder mehrere bekannte Stabilisatoren sind Percarbonsäurelösungen zugänglich, welche nach Kontakt mit Edelstahl noch den SADT-Test (self accelerated decomposition test; UN-Richtlinien zur UN-Klasse 5.2; siehe Orange Book/Transport of Dangerous Goods; Tests and Criteria, Part II, Section 4, 1990, pages 205-209) von _{"}mindestens 60 °C" erfüllen, so daß die Voraussetzungen für eine Transportgenehmigung von Percarbonsäurelösungen in Edelstahlbehältern gegeben sind. Anhand der nachfolgenden Beispiele wird die Erfindung weiter erläutert.

### Beispiele 1 bis 6

Dauer der Gleichgewichtseinstellung bei der Herstellung einer 5 gew.-%igen wäßrigen Peressigsäure bei Verwendung von 1 Gew.-% unterschiedlicher Katalysatoren, bezogen auf das Reaktionsgemisch.

Eine aus 275 g 50 Gew.-% Wasserstoffperoxid, 60 g Essigsäure, 160 g voll entsalztem Wasser und 5 g Katalysator hergestellte Mischung wurde bei 20 °C gehalten und die Zunahme des Peressigsäuregehalts mit der Zeit wurde durch Redoxtitration verfolgt, bis der Peressigsäuregehalt konstant blieb, d.h., das Gleichgewicht erreicht war. Dazu wurden Proben genommen, das darin enthaltene Wasserstoffperoxid durch rasche Titration mit Ce(IV)sulfat und Ferroin-Indikator umgesetzt, unmittelbar anschließend mit einem Überschuß Kaliumjodid versetzt und das durch Reaktion mit Peressigsäure freigesetzte Jod mit Thiosulfat und Stärkeindikator titriert.

Die Zunahme der Peressigsäurekonzentration wurde nach einer Kinetik erster Ordnung ausgewertet. Dazu wurde ln[(c_{G}-c)/c_{G}] gegen die Reaktionszeit t aufgetragen, wobei c_{G} die Peressigsäure-Konzentration im Gleichgewicht und c die Peressigsäure-Konzentration zum Zeitpunkt t ist. Die Auftragung ergibt eine Gerade, aus deren Steigung die in Tabelle 1 zusammengestellten Zeiten bis zum Erreichen von 88 % der Gleichgewichtskonzentration berechnet wurde.

**Tabelle 1**

| Beispiel | Katalysator | Zeit bis zum Erreichen von 88 % der Peressigsäure-Gleichgewichtskonzentation |
|---|---|---|
| 1 | H₂SO₄ | 24 h |
| 2 | HNO₃ | 40 h |
| 3 | H₃PO₄ | 125 h |
| 4 | H₄P₂O₇ | 83 h |
| 5 | meta-H₃P₃O₉ | 263 h |
| 6 | Polyphosphorsäure | 47 h |

Beispiel 6 (erfindungsgemäß) zeigt im Vergleich zu den Beispielen 1 bis 5 (=nicht erfindungsgemäß), daß Polyphosphorsäure eine unerwartet hohe katalytische Aktivität für die Einstellung des Gleichgewichts zwischen Peressigsäure, Essigsäure, Wasserstoffperoxid und Wasser zeigt, die der Aktivität der starken Mineralsäure Salpetersäure (Beispiel 2) gleichkommt. Die Wirkung von Polyphosphorsäure ist deutlich höher als die Wirkung der ortho-Phosphorsäure (Beispiel 3), meta-Phosphorsäure (Beispiel 5) und Pyrophosphorsäure (Beispiel 4).

Die erfindungsgemäß hergestellten Peressigsäuremischungen besitzen deshalb gegenüber den in JP 4 243 861 offenbarten Pyrophosphorsäure-haltigen Peressigsäuremischungen den Vorteil, daß sie unter den gleichen Reaktionsbedingungen schneller oder mit kleineren Mengen an Mineralsäure und somit wirtschaftlicher hergestellt werden können. Polyphosphorsäure ist überdies deutlich preiswerter als Pyrophosphorsäure.

### Beispiele 7 bis 11

### Zersetzung von 5 Gew.-% wäßriger Peressigsäure durch Kontakt mit V2A-Stahl:

Eine Mischung aus 550 g 50 Gew.-% H₂O₂, 120 g Essigsäure, 320 g voll entsalztem Wasser und 0,5 g Dipicolinsäure wurde mit der in Tabelle 2 angeführten Menge an Mineralsäure als Katalysator versetzt und bis zur Einstellung des Gleichgewichts stehen gelassen. Ein V2A-Edelstahlblech mit 150 cm² Oberfläche wurde in diese Gleichgewichtsmischung eingetaucht und die Mischung 13 Tage bei 20 °C gerührt. Anschließend wurden jeweils 100 ml der Mischung auf 60 °C erhitzt und die durch die Zersetzung der Peressigsäure gebildete Gasmenge volumetrisch bestimmt. Tabelle 2 faßt die Resultate zusammen. In Vergleichsversuchen ohne Kontakt mit Edelstahl wurden für die Peressigsäurelösungen aus Beispiel 7 bis 11 unter gleichen Bedingungen Zersetzungsraten von 0,4 bis 0,8 ml/h gefunden.

**Tabelle 2**

| Beispiel | Zugesetzte Mineralsäure in Gew.-% | Durch Peressigsäure-Zersetzung gebildete Gasmenge in ml/h |
|---|---|---|
| 7 | 1 % H₂SO₄ | 14 |
| 8 | 2 % H₂SO₄ | 40 |
| 9 | 1 % H₂SO₄ + 1 % H₃PO₄ | 2,8 |
| 10 | 1 % Polyphosphorsäure | 0,4 |
| 11 | 2 % Polyphosphorsäure | 0,4 |

Die erfindungsgemäßen Peressigsäurelösungen (Beispiele 10 und 11) weisen demnach nach einem längeren Kontakt mit Edelstahl die gleiche Zersetzungsrate auf wie Lösungen, welche nie mit Edelstahl in Berührung kamen.

## Patentansprüche

1. Verfahren zur Herstellung einer Percarbonsäurelösung mit verbesserter Stabilität im Kontakt mit Edelstahl, umfassend Umsetzung einer Carbonsäure mit 1 bis 12 C-Atomen und einer oder zwei Carboxylgruppen mit Wasserstoffperoxid in Gegenwart eines Lösungsmittelsystems und eines sauren Katalysators bei 0 bis 70 °C,
dadurch gekennzeichnet,
daß man als Katalysator Polyphosphorsäure der Formel Hₙ₊₂PₙO₃ₙ₊₁, worin n für den mittleren Kondensationsgrad, der gleich oder größer 2,3 ist, steht, in einer Menge von 0,2 bis 10 Gew.-%, bezogen auf die Percarbonsäurelösung, verwendet.

2. Verfahren nach Anspruch 1,
dadurch gekennzeichnet,
daß man eine Polyphosphorsäure mit einem mittleren Kondensationsgrad von n gleich oder größer 3 verwendet.

3. Verfahren nach Anspruch 1 oder 2,
dadurch gekennzeichnet,
daß man 0,5 bis 5 Gew.-% Polyphosphorsäure, bezogen auf die Percarbonsäurelösung, verwendet.

4. Verfahren nach einem der Ansprüche 1 bis 3,
dadurch gekennzeichnet,
daß man Wasserstoffperoxid in Form einer wäßrigen Wasserstoffperoxidlösung mit einem H₂O₂-Gehalt zwischen 5 und 85 Gew.-% einsetzt.

5. Verfahren nach einem der Ansprüche 1 bis 4,
dadurch gekennzeichnet,
daß das Lösungsmittelsystem wäßrig ist und außer der der Percarbonsäure zugrundeliegenden Carbonsäure keine organischen Lösungsmittel enthält.

6. Verfahren nach einem oder mehreren der Ansprüche 1 bis 5,
dadurch gekennzeichnet,
daß man den Polyphosphorsäure-Katalysator vor der Umsetzung entweder dem Wasserstoffperoxid oder der Carbonsäure zufügt und die Umsetzung durch Mischen des Katalysator enthaltenden Reaktionspartners mit dem anderen Reaktionspartner startet.

7. Verfahren nach einem oder mehreren der Ansprüche 1 bis 6,
dadurch gekennzeichnet,
daß man eine aliphatische Monocarbonsäure mit 1 bis 6 C-Atomen, insbesondere Essigsäure, oder eine α,ω-Dicarbonsäure mit 4 bis 6 C-Atomen mit einer wäßrigen Wasserstoffperoxidlösung bis zur Gleichgewichtseinstellung umsetzt.

8. Verfahren nach einem oder mehreren der Ansprüche 1 bis 7,
dadurch gekennzeichnet,
daß man vor der Umsetzung einem der Reaktionspartner oder Bestandteilen des Lösungsmittelsystems oder während oder nach der Umsetzung dem Reaktionsgemisch einen oder mehrere Stabilisatoren aus der Reihe der (a) Chelatkomplexbildner auf der Basis von Hydroxy- und Aminophosphonsäuren, Amino- und Hydroxycarbonsäuren, N-heterocyclischen Carbonsäuren sowie Salzen der genannten Säuren, (b) Pyrophosphorsäure und deren Salzen, (c) Radikalfänger auf der Basis alkylierter Hydroxyaromaten und (d) Zinnverbindungen zusetzt, vorzugsweise in einer Gesamtmenge von bis zu 0,1 Gew.-%.

## Claims

1. Process for the production of a percarboxylic acid solution having improved stability in contact with stainless steel, comprising the reaction of a carboxylic acid having 1 to 12 C atoms and one or two carboxyl groups with hydrogen peroxide in the presence of a solvent system and an acid catalyst at 0 to 70°C,
characterised in that
the catalyst used is polyphosphoric acid of the formula Hₙ₊₂PₙO₃ₙ₊₁, in which n denotes the average degree of condensation, which is greater than or equal to 2.3, in a quantity of 0.2 to 10 wt.%, relative to the percarboxylic acid solution.

2. Process according to claim 1,
characterised in that
a polyphosphoric acid having an average degree of condensation n of greater than or equal to 3 is used.

3. Process according to claim 1 or 2,
characterised in that
0.5 to 5 wt.% of polyphosphoric acid, relative to the percarboxylic acid solution, is used.

4. Process according to one of claims 1 to 3,
characterised in that
hydrogen peroxide is used in the form of an aqueous hydrogen peroxide solution having an H₂O₂ content of between 5 and 85 wt.%.

5. Process according to one of claims 1 to 4,
characterised in that
the solvent system is aqueous and, apart from the carboxylic acid on which the percarboxylic acid is based, contains no organic solvents.

6. Process according to one or more of claims 1 to 5,
characterised in that
the polyphosphoric acid catalyst is added before the reaction either to the hydrogen peroxide or to the carboxylic acid and the reaction is performed by mixing the reactant containing the catalyst with the other reactant.

7. Process according to one or more of claims 1 to 6,
characterised in that
an aliphatic monocarboxylic acid having 1 to 6 C atoms, in particular acetic acid, or an α,ω-dicarboxylic acid having 4 to 6 C atoms is reacted with an aqueous hydrogen peroxide solution until an equilibrium is established.

8. Process according to one or more of claims 1 to 7,
characterised in that
one or more stabilisers from the series of (a) chelate complexing agents based on hydroxy- and aminophosphonic acids, amino- and hydroxycarboxylic acids, N-heterocyclic carboxylic acids and salts of the stated acids, (b) pyrophosphoric acid and the salts thereof, (c) free-radical scavengers based on alkylated hydroxyaromatics and (d) tin compounds are added before the reaction to one of the reactants or constituents of the solvent system or during or after the reaction to the reaction mixture, preferably in a total quantity of up to 0.1 wt.%.

## Revendications

1. Procédé de préparation d'une solution d'acide percarboxylique de stabilité améliorée en contact avec de l'acier fin, consistant à faire réagir un acide carboxylique ayant 1 à 12 atomes de carbone et un ou deux groupes carboxyle avec du peroxyde d'hydrogène en présence d'un système de solvants et d'un catalyseur acide à une température de 0 à 70°C,
caractérisé en ce qu'on utilise comme catalyseur de l'acide polyphosphorique de formule Hₙ₊₂PₙO₃ₙ₊₁, dans laquelle n désigne le degré de condensation moyen qui est égal ou supérieur à 2,3, en quantité de 0,2 à 10% en poids par rapport à la solution d'acide percarboxylique.

2. Procédé selon la revendication 1,
caractérisé en ce qu'on utilise un acide polyphosphorique ayant un degré de condensation moyen n égal ou supérieur à 3.

3. Procédé selon la revendication 1 ou 2,
caractérisé en ce qu'on utilise 0,5 à 5% en poids d'acide polyphosphorique par rapport à la solution d'acide percarboxylique.

4. Procédé selon l'une quelconque des revendications 1 à 3,
caractérisé en ce qu'on utilise du peroxyde d'hydrogène sous la forme d'une solution aqueuse de peroxyde d'hydrogène ayant une teneur en H₂O₂ entre 5 et 85% en poids.

5. Procédé selon l'une quelconque des revendications 1 à 4,
caractérisé en ce que le système de solvants est aqueux et ne contient aucun solvant organique en dehors de l'acide carboxylique qui est à la base de l'acide percarboxylique.

6. Procédé selon une ou plusieurs des revendications 1 à 5,
caractérisé en ce qu'on ajoute le catalyseur d'acide polyphosphorique, avant la réaction, au peroxyde d'hydrogène ou à l'acide carboxylique et la réaction est amorcée par mélange du partenaire réactionnel contenant le catalyseur à l'autre partenaire réactionnel.

7. Procédé selon une ou plusieurs des revendications 1 à 6,
caractérisé en ce qu'on fait réagir un acide monocarboxylique aliphatique ayant 1 à 6 atomes de carbone ou un acide α,ω-dicarboxylique ayant 4 à 6 atomes de carbone avec une solution aqueuse de peroxyde d'hydrogène jusqu'à l'équilibre.

8. Procédé selon une ou plusieurs des revendications 1 à 7,
caractérisé en ce qu'on ajoute, avant la réaction, à l'un des partenaires réactionnels ou des composants du système de solvants ou, pendant ou après la réaction, au mélange réactionnel un ou plusieurs stabilisateurs choisis dans la série comprenant (a) des adjuvants complexes chélatants à base d'acides hydroxy- et aminophosphoniques, d'acides amino- et hydroxycarboxyliques, d'acides carboxyliques N-hétérocycliques et de sels des acides mentionnés, (b) l'acide pyrophosphorique et ses sels, (c) des absorbeurs de radicaux à base d'hydroxyaromatiques alkylés et (d) des composés d'étain, de préférence en quantité totale jusqu'à 0,1% en poids.
